# EUROPÄISCHE PATENTSCHRIFT

(11) **EP 1 132 107 B1**
(45) Veröffentlichungstag und Bekanntmachung des Hinweises auf die Patenterteilung: **08.12.2004**
(21) Anmeldenummer: 01105878.1
(22) Anmeldetag: 09.03.2001
(51) Int. Cl.: A61M 39/10

(54) **Konnektor mit innerem Verschiebeelement**
Connector with a displaceable internal element
Connecteur avec un élément interne mobile

(30) Priorität: 10.03.2000 DE 10011724
(43) Veröffentlichungstag der Anmeldung: 12.09.2001
(73) Patentinhaber: Fresenius Medical Care Deutschland GmbH, 61352 Bad Homburg (DE)
(72) Erfinder: Pott, Harald, Dr., 42499 Hückeswagen (DE); Schmidt, Helmut, 66649 Oberthal (DE)
(74) Vertreter: Laufhütte, Dieter, Dr.-Ing.

(56) Entgegenhaltungen:
- EP-A- 0 569 030
- EP-A- 0 798 013
- US-A- 5 848 994

## Beschreibung

Die Erfindung betrifft einen Konnektor mit innenliegendem Verschiebeelement, welches in einem ersten Zustand den Durchfluß einer Flüssigkeit aus einem Beutel in ein Schlauchsystem verhindert und nach Verschieben den Durchfluß erst ermöglicht.

Es ist im Stand der Technik bekannt, aus steril verpackten Systemen den Durchfluß durch einen Schlauch erst bei Anwendung zu ermöglichen. In der EP 0 038 355 A1 befindet sich im Schlauchinnern ein Abbrechpin, der - um den Durchfluß freizugeben - abgebrochen wird und sich dann quer in dem Schlauch verklemmen soll. Nachteilig an dieser Konstruktion ist die Möglichkeit, daß winzige Bruchpartikel in die Lösung gelangen können und dadurch an den Applikationsort, was insbesondere bei Intraperitoneallösungen oder Infusionslösungen bei den Patienten unangenehme Begleiterscheinungen hervorruft. Auch sind Durchstechmembrane bekannt, die die gleichen Nachteile aufweisen.

Auch sind innenliegende Verschiebeelemente in Adaptoren aus der EP 0 798 013 A1 bekannt, wo das Verschiebeelement reversibel in dem Adapter gelagert ist.

Durch die Rückstellkraft einer innenliegenden Feder wird das Verschiebeelement ohne äußere Einwirkung immer wieder in die Ausgangsposition zurückgeschoben. Der Aufbau eines derartigen Verschiebeelementes ist sehr aufwendig und teuer herzustellen und kann deshalb in Einmalsystemen nicht zum Einsatz kommen.

Ein Dokument, das die Merkmale des Oberbegriffs des Anspruchs 1 offenbart, ist das EP 0 569 030.

Es ist Aufgabe der vorliegenden Erfindung, einen wegwerfbaren Konnektor vorzugsweise zum Anschluß an medizinische Geräte zu schaffen, der einfach und preisgünstig herzustellen ist, und der den Durchfluß in einem ersten Zustand verhindert und nach irreversiblem Überführen in einen zweiten Zustand den Durchfluß ermöglicht. Die Aufgabe wird durch die Merkmale des Anspruchs 1 gelöst.

In medizinischen Behandlungssystemen werden oft Lösungen verwendet, beispielsweise Desinfektionslösungen, Infusionslösungen oder während einer Nierenersatzbehandlung sog. Dialyselösungen. Diese Lösungen werden vorzugsweise steril verpackt in Containern oder flexiblen Beutel geliefert, oder in den Vorratsbehältern vor Ort hergestellt. Die Vorratsbehälter werden über einen Konnektor an das Behandlungssystem angeschlossen, so daß die Lösung während der Behandlung zur Verfügung steht. Dabei ist es von besonderem Interesse, daß der Konnektor anfangs verschlossen ist, bis die Lösung zum Einsatzort kommt, um dann in einen geöffneten Zustand überführt zu werden. Um vorzeitiges, versehentliches Auslaufen der Lösung zu verhindern, sollte eine manuelle Öffnung durch den Bediener bei üblicher Behandlung vermieden werden.

Aus diesem Grund sind Konnektoren oder Adaptoren entwickelt worden, die nach dem Anschluß oder dem Zusammenbau eines Schlauchsystems erst manuell geöffnet werden. Der erfindungsgemäße Konnektor gehört ebenfalls dieser Gattung an, weist jedoch den Vorteil auf, daß mit gleichzeitigem Anschluß an die Maschine der Durchfluß geöffnet wird, während vorher eine Abdichtung gegen die Umgebung gewährleistet wurde. Diese Wirkung wird durch einen inneren Kolben erzielt, der durch ein entsprechendes Gegenstück an der Maschine in einem zylindrischen Gehäuse genau einmal axial verschoben werden kann. Beim Anschluß an die Maschine wird ein innenliegender, verschiebbarer Kolben in den vorderen Teil des Konnektors geschoben und damit die Durchflußöffnung zur Fluidweiterführung freigelegt.

Vorzugsweise besteht der Konnektor aus Kunststoff, besonders ausgestaltet aus zwei verschiedenen Kunststoffmaterialien. Während der äußere Konnektorteil aus Polypropylen besteht, insbesondere bei Verbindung mit anderen Polyolefinmaterialien, sollte der verschiebbare Innenteil aus Silikonkautschuk bestehen. Dadurch ist der gesamte Konnektor auch in Verbindung mit dem Schlauchsystem ein Wegwerfteil, welches im Spritzgußverfahren kostengünstig und durch die einfache Montage der zwei Bauteile konstruktiv einfach herstellbar ist. Durch die geringe Bauteilanzahl ist natürlich auch die Fehleranfälligkeit extrem herabgesetzt.

Zusätzlich zu dem einfachen Handling für den Anwender, das keine zusätzliche Handbewegung, wie Abbrechen eines Pins, Verschrauben oder Drehen verlangt, wird auch sicher verhindert, daß keine Partikel in das weiterführende Schlauchsystem gelangen.

Indem sich kein Gegenstand in undefinierter Weise in die weiterführende Leitung legt, ist stets der gleiche Durchfluß gewährleistet, da der innenliegende Kolben durch seinen Anschlag immer in die gleiche, identische Position geschoben wird und die definierte Durchflußöffnung freigibt. Die Durchflußöffnung kann durch ein oder mehrere seitliche Löcher gebildet werden, die sich in einem ersten, geschlossenen Zustand in einem Abschnitt des Konnektors befinden. Dieser weist ein derart enges Lumen auf, daß die Durchflußöffnungen dichtend anliegen oder flußauf dieser verjüngten Stelle liegen, an der das Verschiebeelement dichtend an der Konnektorinnenseite anliegt. In einem zweiten, geöffneten Zustand wird das Verschiebeelement in einen Bereich des Konnektors geschoben, der einen größeren Innendurchmesser aufweist. Dadurch kommen die Durchflußöffnungen in einen der verjüngten Stelle stromab liegenden Bereich und sind für den Durchfluß freigelegt.

Weitere Einzelheiten und Vorteile der Erfindung ergeben sich aus der sich anschließenden Beschreibung bevorzugter Ausgestaltungen der Erfindung.

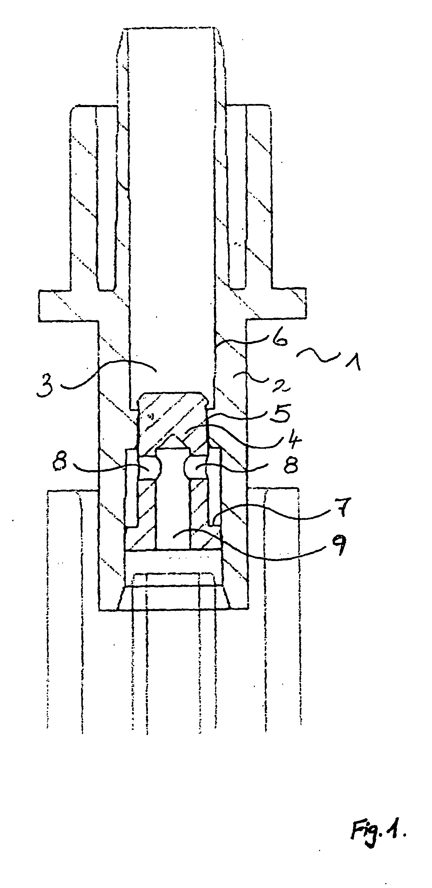

Figur 1 zeigt einen gemäß einer erfindungsgemäßen Ausführungsform gestalteten Konnektor 1 mit geschlossenem Auslaß. Der zylindrische Körper 2 umfängt ein hohles Lumen 3, in welchem ein Verschiebeelement 4 beweglich gelagert ist. Dabei weist das Lumen 3 mindestens einen im Durchmesser verjüngten Lumenbereich 5 und einen davon beabstandeten Lumenbereich 6 auf. Vorzugsweise dient der verjüngte Lumenbereich 5 gleichzeitig als Anschlagfläche für den Anschlag 7. Des weiteren weist das Verschiebeelement 4 mindestens eine Durchflußöffnung 8 auf. In der gezeigten Ausführungsform liegt die Durchflußöffnung 8 oberhalb der verjüngten Stelle 5. Denkbar ist jedoch auch eine Ausführungsform, die die Öffnung 8 in Höhe der verjüngten Stelle 5 zeigt, so daß die Innenseite 5 des Konnektors 1 gleichzeitig die Abdichtung der Durchflußöffnung 8 übernimmt. Zusätzliche Dichtungen insbesondere O-Ring-Dichtungen sind an der verjüngten Stelle denkbar.

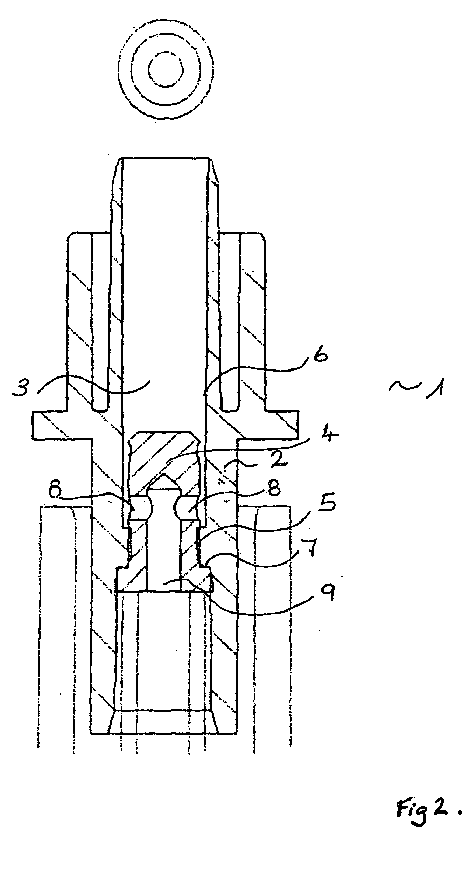

Figur 2 zeigt den erfindungsgemäßen Konnektor 1 im für den Durchfluß geöffneten Zustand. Das innenliegende Verschiebeelement 4 ist in eine zweite Position verschoben. Es werden die gleichen Bezugszeichen verwendet. In dieser Figur liegt der Anschlag 7 an der Anschlagfläche an und verhindert so ein Weiterrutschen des Verschiebeelementes 4. Die Durchflußöffnung 8 befindet sich stromab der verjüngten Stelle 5 im Lumenteil 3, so daß ein Durchfluß durch den Spalt erfolgt, der zwischen der Innenwand 6 des Konnektors 1 und dem Verschiebeelement 4 gebildet ist. Um den Durchfluß zu ermöglichen, tritt die Lösung in den inneren Hohlraum 9 des Verschiebeelementes ein, um durch die Durchflußöffnung 8 nach außen wieder auszutreten. Die Erfindung ist jedoch nicht auf diese Ausführungsform beschränkt.

## Patentansprüche

1. Konnektor (1) mit Schlauchanschlüssen am proximalen und distalen Ende, mit einem Verschiebeelement (4), welches in axialer Richtung des Konnektors (1) bewegbar ist und ein inneres durchströmbares Lumen (9) aufweist mit mindestens einer Auslauföffnung und mindestens einer in einer ersten Position dichten Durchflußöffnung (8) , wobei das Verschiebeelement (4) einen Anschlag (7) aufweist, um eine zweite feste Position zu definieren, in der mindestens eine Durchflußöffnung (8) für den Durchfluß geöffnet ist,
**dadurch gekennzeichnet,**
**daß** das Verschiebeelement (4) vollständig im Inneren des als zylindrischer Körper ausgestalteten Konnektors (1) liegt und daß es durch Anschluß an eine Maschine durch diese derart verschiebbar ist, daß die mindestens eine Durchflußöffnung (8) zur Fluidweiterführung freigelegt ist.

2. Konnektor nach Anspruch 1, **dadurch gekennzeichnet, daß** er aus Kunststoff hergestellt ist.

3. Konnektor nach einem der Ansprüche 1 und 2, **dadurch gekennzeichnet, daß** verschiedene Teile des Konnektors (1) aus unterschiedlichen Kunststoffen bestehen.

4. Konnektor nach einem der Ansprüche 1 bis 3, **dadurch gekennzeichnet, daß** der äußere Teil des Konnektors (1) aus Polypropylen besteht, während das Verschiebeelement (4) aus Silikonkautschuk besteht.

5. Konnektor nach einem der Ansprüche 1 bis 4, **dadurch gekennzeichnet, daß** er ein Einmalartikel oder Teil eines Einmalartikels ist.

6. Medizinisches Schlauchsystem mit einem Konnektor nach Anspruch 1, **dadurch gekennzeichnet, daß** es zum Anschluß an ein medizinisches Gerät dient.

7. Medizinisches Schlauchsystem nach Anspruch 6, **dadurch gekennzeichnet, daß** das medizinische Gerät ein Nierendialysegerät ist.

## Claims

1. Connector (1) with tubular attachments at the proximal and distal ends, with a displacement element (4) which can be moved in axial direction of the connector (1) and has an inner lumen (9) through which a flow can pass, with at least one discharge outlet and at least one through-flow opening (8) tight in a first position, the displacement element (4) having a stop (7) in order to define a second fixed position in which at least one through-flow opening (8) is opened for the through-flow,
**characterized in that**
the displacement element (4) lies wholly inside the connector (1) which is designed as a cylindrical body and **in that**, by connection to a machine, it can be displaced by this, such that the at least one through-flow opening (8) is uncovered to conduct the fluid further.

2. Connector according to claim 1, **characterized in that** it is made from plastic.

3. Connector according to one of claims 1 and 2, **characterized in that** different parts of the connector (1) are made from different plastics.

4. Connector according to one of claims 1 to 3, **characterized in that** the outer part of the connector (1) is made from polypropylene, while the displacement element (4) consists of silicone rubber.

5. Connector according to one of claims 1 to 4, **characterized in that** it is a disposable article or part of a disposable article.

6. Medical tube system with a connector according to claim 1, **characterized in that** it serves for connection to a medical apparatus.

7. Medical tube system according to claim 6, **characterized in that** the medical apparatus is a kidney dialysis apparatus.

## Revendications

1. Connecteur (1) avec des branchements de flexible sur l'extrémité proximale et l'extrémité distale, avec un élément coulissant (4), qui peut être déplacé dans le sens axial du connecteur (1) et présente un lumen (9) intérieur pouvant être traversé avec au moins une ouverture de sortie et au moins une ouverture d'écoulement (8) étanche dans une première position, l'élément coulissant (4) présentant une butée (7), afin de définir une seconde position fixe dans laquelle au moins une ouverture d'écoulement (8) est ouverte pour l'écoulement,
**caractérisé en ce que**
l'élément coulissant (4) est situé entièrement à l'intérieur du connecteur (1) conçu comme un corps cylindrique et **en ce qu'**il peut être déplacé par raccordement à une machine à travers cette machine de telle sorte que la au moins une ouverture d'écoulement (8) est libérée pour l'acheminement ultérieur du fluide.

2. Connecteur selon la revendication 1, **caractérisé en ce qu'**il est fabriqué à base de matière synthétique.

3. Connecteur selon l'une quelconque des revendications 1 et 2, **caractérisé en ce que** différentes parties du connecteur (1) sont à base de différentes matières synthétiques.

4. Connecteur selon l'une quelconque des revendications 1 à 3, **caractérisé en ce que** la partie extérieure du connecteur (1) est à base de polypropylène, alors que la partie coulissante (4) est à base de caoutchouc de silicone.

5. Connecteur selon l'une quelconque des revendications 1 à 4, **caractérisé en ce qu'**il s'agit d'un article à usage unique ou une partie d'un article à usage unique.

6. Système de flexible médical avec un connecteur selon la revendication 1, **caractérisé en ce qu'**il sert au raccordement à un appareil médical.

7. Système de flexible médical selon la revendication 6, **caractérisé en ce que** l'appareil médical est un appareil de dialyse pour les reins.
